# EUROPÄISCHE PATENTSCHRIFT

(11) **EP 3 210 533 B1**
(45) Veröffentlichungstag und Bekanntmachung des Hinweises auf die Patenterteilung: **11.12.2019**
(21) Anmeldenummer: 17157090.6
(22) Anmeldetag: 21.02.2017
(51) Int. Cl.: A61B 5/053, A61B 5/00

(54) **VORRICHTUNG UND METHODE ZUM MESSEN EINES ELEKTRISCHEN HAUTWIDERSTANDES**
DEVICE AND METHOD FOR MEASURING A CUTANEOUS ELECTRIC RESISTANCE
DISPOSITIF ET PROCÉDÉ DE MESURE DE LA RÉSISTANCE ÉLECTRIQUE DE LA PEAU

(30) Priorität: 23.02.2016 AT 501242016
(43) Veröffentlichungstag der Anmeldung: 30.08.2017
(73) Patentinhaber: Peutler, Peter, 8054 Seiersberg-Pirka (AT)
(72) Erfinder: Peutler, Peter, 8054 Seiersberg-Pirka (AT)
(74) Vertreter: Gibler & Poth Patentanwälte KG

(56) Entgegenhaltungen:
- EP-B1- 1 171 030
- WO-A1-2011/127306
- CN-U- 204 813 893
- DE-A1- 2 810 344
- US-A1- 2014 238 153
- US-A1- 2016 029 891

## Beschreibung

Die Erfindung betrifft eine Vorrichtung zum Messen eines elektrischen Hautwiderstandes gemäß dem Oberbegriff des Patentanspruches 1.

Es sind Vorrichtungen zum Messen des elektrischen Hautwiderstandes bekannt, welche insbesondere bei alternativmedizinischen Verfahren wie Elektroakkupunktur eingesetzt werden. Dabei wird die Vorrichtung mit der Messelektrode von einem Benutzer mit vielen vorgegebenen Messstellen eines Probanden in Kontakt gebracht, wobei der Proband an einer vorgegebenen Bezugsstelle mit einer Bezugselektrode in Kontakt ist. Durch Anlegen eines Messstromes zwischen der Messelektrode und der Bezugselektrode kann der elektrische Widerstand zwischen diesen beiden Elektroden bestimmt werden, und in weiterer Folge aus diesem elektrischen Widerstand der Hautwiderstand an der vorgegebenen Messstelle bestimmt werden.

Da oftmals eine Vielzahl an Messungen an verschiedenen vorgegebenen Messstellen notwenig sind und die vorgegebenen Messstellen oftmals lediglich eine kleine Ausdehnung aufweisen, wird die Messelektrode nicht am Körper des Probanden befestigt, sondern die Vorrichtung mit der Messelektrode wird von einem Benutzer, beispielsweise einem Therapeuten, mit einem Handgriff ähnlich wie ein Stift gehalten. Hierbei hat sich gezeigt, dass die Anpresskraft, mit welchem die Messelektrode an die Haut des Probanden gedrückt wird, einen starken Einfluss auf den elektrischen Hautwiderstand hat, weshalb der Benutzer oftmals viel Erfahrung braucht um reproduzierbare Messergebnisse bei annähernd gleicher Anpresskraft zu erhalten. Weiters besteht dadurch das Problem, dass der Benutzer durch die Variation der Anpresskraft unterbewusst stark auf die erhaltenen Messergebnisse Einfluss nehmen kann.

Hierbei sind Vorrichtungen bekannt, bei welchen die Messelektrode in Längsrichtung des Handgriffes federnd gelagert ist und ein Dehnmessstreifen die auf die Messelektrode wirkende Kraft in Längsrichtung des Handgriffes misst. Durch eine derartige Vorrichtung kann es dem Benutzer vereinfacht werden, die Anpresskraft in einem vorgegebenen Bereich zu halten, und dadurch die Reproduzierbarkeit der Messergebnisse zu verbessern.

Aus der EP 1 171 030 B1 ist eine Vorrichtung zum Messen eines elektrischen Hautwiderstandes bekannt. Die Vorrichtung weist einen stiftförmigen Handgriff auf, wobei an einem Ende des Handgriffes eine Messelektrode angeordnet ist. Die Messelektrode ist mit einem Kraftaufnehmer wirkverbunden, welcher Drücke auf die Messelektrode in Längsrichtung des Handgriffes aufnimmt.

Aus der US 2016/0029891 A1 ist ein Verfahren zur Überwachung eines Patienten in einem Krankenhaus bekannt.

Aus der WO 2011/127306 A1 ist ein Kraftsensor für medizinische Zwecke bekannt. Der Kraftsensor weist hierbei eine zweidimensionale Anordnung einzelner Kraftsensoren in einem elastischen Körper auf, welche die Lasten an den einzelnen Punkten der Anordnung bestimmen.

Aus der DE 281 03 44 A1 ist eine Vorrichtung zur Gleichstromwiderstandsmessung an einem Menschen bekannt. Die Vorrichtung weist ein stiftförmiges Gehäuse auf, in welchem ein axial gegen die Kraft einer Feder verschiebbarer Stift mit einer Punktelektrode gelagert ist.

Aus der US 2014/0238153 A1 ist ein flächiger Verformungssensor bekannt, welcher in der Lage ist Verformungen einer elastischen Fläche aufzunehmen.

Aus der CN 204813893 A ist ein flächiger Sensor bekannt, welcher die Krafteinwirkung auf einem Fuß eines Probanden für medizinische Zwecke erfasst.

Nachteilig daran ist, dass derartige Vorrichtungen in einer vorgegebenen Ausrichtung zu der Haut orientiert sein müssen, damit ein Messfehler vermieden werden kann. In der Praxis hat sich diese vorgegebene Orientierung der Vorrichtung zur Haut an der Messstelle allerdings oft als unpraktikabel herausgestellt, da oft die Benutzer aus Bequemlichkeit oder Unerfahrenheit die Vorrichtung falsch halten und dadurch Messfehler entstehen, insbesondere da die zur Messung vorgesehenen Messstellen oftmals an den Händen und Füßen der Probanden sind, welche eine komplexe Topografie aufweisen. Durch diese komplexe Topografie wird es wesentlich erschwert, die Messelektrode mit einer vorgegebenen Ausrichtung mit verschiedenen Messstellen der Haut zu kontaktieren. Weiters weisen unterschiedliche Benutzer oftmals eine unterschiedliche Handhaltung der Vorrichtung auf, wodurch die Messergebnisse zweier Benutzer nur bedingt verglichen werden können.

Aufgabe der Erfindung ist es daher eine Vorrichtung zum Messen eines elektrischen Hautwiderstandes der eingangs genannten Art anzugeben, mit welcher die genannten Nachteile vermieden werden können, mit welcher die Genauigkeit und Reproduzierbarkeit der Messung des Hautwiderstandes verbessert werden kann und welche einfach in der Handhabung ist.

Erfindungsgemäß wird dies durch die Merkmale des Patentanspruches 1 erreicht.

Dadurch ergibt sich der Vorteil, dass durch die Handhabung der Vorrichtung durch den Benutzer verursachte Messfehler bei der Messung des Hautwiderstandes verringert werden können, wodurch die Genauigkeit und Reproduzierbarkeit der Messung des Hautwiderstandes verbessert wird und gleichzeitig die Handhabung der Vorrichtung vereinfacht wird. Hierbei hat sich gezeigt, dass durch die Verwendung eines in wenigstens zwei Koordinaten messenden Kraftaufnehmers der Benutzer wesentlich freier bei der Handhabe der Vorrichtung, insbesondere des Winkels welcher die Vorrichtung zu der Haut an der vorgegebenen Messstelle einnimmt, ist, und dennoch die Anpresskraft genau bestimmbar ist, wodurch reproduzierbar ein elektrischer Hautwiderstand bei einer Referenzanpresskraft bestimmt werden kann. Dadurch kann auch wenig erfahrenes Personal reproduzierbare Messungen des Hautwiderstandes durchführen. Weiters können von unterschiedlichen Benutzern durchgeführten Messreihen eines Probanden zuverlässig miteinander verglichen werden, um beispielsweise eine mehrere Monate dauernde Abfolge von Messreihen durchzuführen.

Weiters ist ein Verfahren zum Messen eines elektrischen Hautwiderstandes eines Probanden gemäß dem Patentanspruch 8 vorgesehen.

Aufgabe der Erfindung ist es daher weiters ein Verfahren der eingangs genannten Art anzugeben, mit welcher die genannten Nachteile vermieden werden können, mit welcher die Genauigkeit und Reproduzierbarkeit der Messung des Hautwiderstandes verbessert werden kann und welche eine einfache Handhabung ermöglicht.

Erfindungsgemäß wird dies durch die Merkmale des Patentanspruches 8 erreicht.

Die Vorteile des Verfahrens entsprechen den Vorteilen der Vorrichtung.

Die Unteransprüche betreffen weitere vorteilhafte Ausgestaltungen der Erfindung.

Ausdrücklich wird hiermit auf den Wortlaut der Patentansprüche Bezug genommen, wodurch die Ansprüche an dieser Stelle durch Bezugnahme in die Beschreibung eingefügt sind und als wörtlich wiedergegeben gelten.

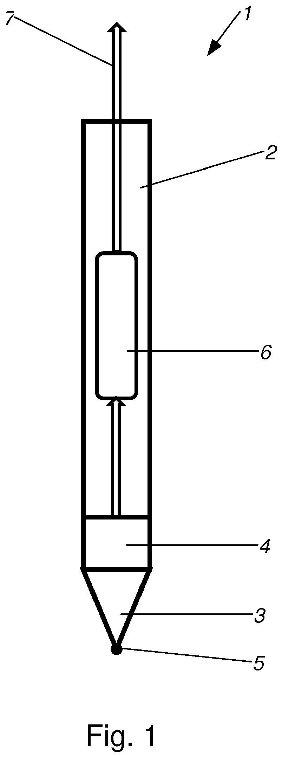

Die Erfindung wird unter Bezugnahme auf die beigeschlossene Zeichnung, in welcher lediglich eine bevorzugte Ausführungsform beispielhaft dargestellt ist, näher beschrieben. Dabei zeigt die Fig. 1 eine bevorzugte Ausführungsform der Vorrichtung.

Die Fig. 1 zeigt eine bevorzugte Ausführungsform einer Vorrichtung 1 zum Messen eines elektrischen Hautwiderstandes, umfassend einen stiftförmigen Handgriff 2, eine an einem Ende des Handgriffes 2 angeordnete im Wesentlichen punktförmige Messelektrode 3 und einen Kraftaufnehmer 4. Die Vorrichtung 1 ist dazu vorgesehen beim Messen des elektrischen Hautwiderstandes an einer vorgegebenen Messstelle verwendet zu werden. Der elektrische Hautwiderstand ist das Maß für den elektrischen Widerstand der Haut an der vorgegebenen Messstelle. Die physikalische Einheit für den elektrischen Hautwiderstand ist das Ohm.

Die Vorrichtung 1 weist eine im Wesentlichen punktförmige Messelektrode 3 auf, welche dazu vorgesehen ist mit der vorgegebenen Messstelle der Haut direkt kontaktiert zu werden, sodass ein elektrischer Messstrom an einer Kontaktfläche der Messelektrode 3 mit der Haut der vorgegebenen Messstelle zwischen dem Körper des Probanden und der Messelektrode 3 fließen kann. Dass die Messelektrode 3 im Wesentlichen punktförmig ist bedeutet, dass die in Kontakt zu der Haut stehende Messelektrode 3 lediglich eine sehr kleine Kontaktfläche zwischen der Messelektrode 3 und der Haut ausbildet, insbesondere dass die Kontaktfläche kleiner 10 mm², besonders bevorzugt kleiner 3 mm², ist. Eine derartige im Wesentlichen punktförmige Messelektroden 3 kann auch als Messspitze bezeichnet werden.

Die Vorrichtung 1 weist weiters einen stiftförmigen Handgriff 2 auf, welcher dazu vorgesehen von dem Benutzer gehalten zu werden. Der Handgriff 2 kann insbesondere die Form eines länglichen Stabes aufweisen, wie es insbesondere bei Schreibstiften üblich ist, sodass die Vorrichtung 1 von dem Benutzer ähnlich wie ein Schreibstift gehalten werden kann. Insbesondere kann die gesamte Vorrichtung 1 eine einem Schreibstift ähnelnde Form aufweisen. Der Handgriff 2 kann insbesondere eine Grifffläche für die Hand des Benutzers aufweisen. Der Handgriff 2, insbesondere die Grifffläche, kann bevorzugt ergonomisch geformt sein. An einem Ende des Handgriffes 2 ist die Messelektrode 3 angeordnet, sodass die Vorrichtung 1 ähnlich handhabbar ist wie ein Schreibstift und dadurch eine intuitive Benutzung durch den Benutzer möglich ist. An einem der Messelektrode 3 gegenüberliegenden Ende des Handgriffes 2 kann ein elektrisches Kabel 7 aus dem Handgriff 2 führen. Hierbei kann ein elektrischer Leiter von der Messelektrode 3 durch den Handgriff 2 in das elektrische Kabel 7 führen.

Weiters weist die Vorrichtung 1 einen Kraftaufnehmer 4 auf. Ein Kraftaufnehmer 4 ist ein Messgerät zum Messen von Kräften. Die von dem Kraftaufnehmer 4 gemessene Kraft kann vom Kraftaufnehmer 4 in ein elektrisches Messsignal gewandelt werden. Die physikalische Einheit für die gemessene Kraft ist das Newton.

Vorgesehen ist, dass der Kraftaufnehmer 4 auf die Messelektrode 3 einwirkende Kräfte in wenigstens zwei Koordinaten misst. Hierbei ist der Kraftaufnehmer 4 derart ausgebildet und angeordnet, dass dieser auf die Messelektrode 3 einwirkende Kräfte in wenigstens zwei Koordinaten misst. Der Kraftaufnehmer 4 kann insbesondere ein mehrachsiger Kraftaufnehmer 4 mit wenigstens zwei unterschiedlichen Messachsen sein. Die Koordinaten können auch als Dimensionen bezeichnet werden. Diese wenigstens zwei Koordinaten können insbesondere zwei, insbesondere orthogonal zueinander stehende, kartesische Raumkoordinaten sein. Diese auf die Messelektrode 3 einwirkende Kraft kann insbesondere im Wesentlichen die Anpresskraft sein, mit welcher der Benutzer die Messelektrode 3 über den Handgriff 2 an die Haut des Probanden anpresst. Die auf die Messelektrode 3 einwirkende Kraft kann weiters auch die Gewichtskraft der Messelektrode 3 selber umfassen, wobei die Anpresskraft insbesondere wesentlich höher sein kann als die Gewichtskraft. Es kann daher vorgesehen sein, die von dem Kraftaufnehmer 4 gemessene Kraft mit der Anpresskraft gleichzusetzen.

Bei einer Variation der Anpresskraft variiert die Kontaktfläche zwischen der Messelektrode 3 und der Haut des Probanden, da die Haut des Probanden flexibel ist, wodurch der elektrische Hautwiderstand verändert wird. Weiters ist der elektrische Hautwiderstand auch von einem auf die Haut einwirkenden Druck abhängig, da durch einen Druck die einzelnen Hautschichten zusammengepresst werden.

Bereits durch die Messung der auf die Messelektrode 3 einwirkende Kraft in lediglich zwei Koordinaten kann die Genauigkeit der Messung bereits wesentlich verbessert werden. Wenn beispielsweise der Kraftaufnehmer 4 die Kraft in einer Längsrichtung des Handgriffes 2 und in einer Richtung normal zu der Längsrichtung des Handgriffes 2 misst, kann der Benutzer den Winkel zwischen der Längsrichtung des Handgriffes 2 und der Flächennormalen, welcher auch als Anstellwinkel bezeichnet werden kann, bereits frei wählen, und muss lediglich darauf achten, die Vorrichtung bei der Benutzung nicht um die Längsrichtung zu drehen. Wenn alternativ der Kraftaufnehmer 4 die Kraft in zwei normal zu der Längsrichtung stehenden Richtung misst, kann der Benutzer die Vorrichtung bereits schräg halten, und muss lediglich darauf achten den Anstellwinkel konstant zu halten. Dadurch kann die Anpresskraft bestimmt werden, wodurch ein elektrischer Hautwiderstand bei einer vorgebbaren Referenzanpresskraft gemessen werden, und der Fehler durch die variierende Anpresskraft gering gehalten werden kann.

Dadurch ergibt sich der Vorteil, dass durch die Handhabung der Vorrichtung 1 durch den Benutzer verursachte Messfehler bei der Messung des Hautwiderstandes verringert werden können, wodurch die Genauigkeit und Reproduzierbarkeit der Messung des Hautwiderstandes verbessert wird und gleichzeitig die Handhabung der Vorrichtung 1 vereinfacht wird. Hierbei hat sich gezeigt, dass durch die Verwendung eines in wenigstens zwei Koordinaten messenden Kraftaufnehmers 4 der Benutzer wesentlich freier bei der Handhabe der Vorrichtung 1, insbesondere des Winkels welcher die Vorrichtung 1 zu der Haut an der vorgegebenen Messstelle einnimmt, ist, und dennoch die Anpresskraft genau bestimmbar ist, wodurch reproduzierbar ein elektrischer Hautwiderstand bei einer Referenzanpresskraft bestimmt werden kann. Dadurch kann auch wenig erfahrenes Personal reproduzierbare Messungen des Hautwiderstandes durchführen. Weiters können von unterschiedlichen Benutzern durchgeführten Messreihen eines Probanden zuverlässig miteinander verglichen werden, um beispielsweise eine mehrere Monate dauernde Abfolge von Messreihen durchzuführen.

Weiters ist ein Verfahren zum Messen des elektrischen Hautwiderstandes des Probanden vorgesehen, wobei eine Bezugselektrode an einer vorgegebenen Bezugsstelle von dem Probanden gehalten oder an dem Probanden befestigt wird, wobei die an einem Ende des stiftförmigen Handgriffes 2 angeordnete im Wesentlichen punktförmige Messelektrode 3 an wenigstens einer vorgegebenen Messstelle mit einer Anpresskraft gegen die Haut des Probanden gedrückt wird, wobei auf die Messelektrode 3 einwirkende Kräfte in wenigsten zwei Koordinaten von dem Kraftaufnehmer 4 gemessen und als Messdaten an eine Auswerteeinheit übermittelt werden, wobei ein Messstrom zwischen der Bezugselektrode und der Messelektrode 3 erzeugt wird, wobei von der Auswerteeinheit anhand einer elektrischen Größe des Messstromes und unter Berücksichtigung der Messdaten des Kraftaufnehmers 4 der elektrische Hautwiderstand eines Probanden bei einer Referenzanpresskraft an der wenigstens einen vorgegebenen Messstelle ermittelt wird.

Bei dem Messverfahren wird ein elektrischer Stromkreis für den Messstrom hergestellt, welcher die Auswerteeinheit, die Bezugselektrode und die Messelektrode umfasst, wobei der schaltungstechnisch zwischen der Bezugselektrode und die Messelektrode angeordnete Proband einen elektrischen Widerstand, den sogenannten Körperwiderstand, darstellt. Der Körperwiderstand kann als Serienschaltung des Hautwiderstandes an der vorgegebenen Bezugsstelle und der vorgegebenen Messstelle sowie des Widerstandes des Strompfades innerhalb des Körpers angesehen werden. Wenn der Hautwiderstand an der vorgegebenen Bezugsstelle sowie der Widerstand des Strompfades innerhalb des Körpers bekannt und/oder vernachlässigbar klein ist, lässt sich der Hautwiderstand an der vorgegebenen Messstelle leicht durch den gemessenen elektrischen Widerstand zwischen der Bezugselektrode und der Messelektrode bestimmen.

Der Messstrom kann insbesondere durch die Auswerteeinheit erzeugt werden, wobei die Auswerteeinheit besonders bevorzugt als Stromquelle für den Messstrom ausgebildet ist.

Die Auswerteeinheit kann mit einer externen Energiequelle, insbesondere einem elektrischen Verteilernetz, verbindbar sein oder über eine interne Energiequelle, insbesondere Batterien oder Akkumulatoren, verfügen.

Der Messstrom kann insbesondere eine konstante und vorgegebene Stromstärke aufweisen.

Alternativ kann der Messstrom insbesondere eine konstante und vorgegebene Spannung aufweisen.

Die Auswerteeinheit misst eine elektrische Größe des Messstromes, wobei die elektrische Größe des Messstromes insbesondere der elektrische Spannungsabfall zwischen der Bezugselektrode und der Messelektrode sein kann. Die Auswerteeinheit kann daher insbesondere einen Spannungsmesser aufweisen.

Alternativ kann die von der Auswerteeinheit gemessene elektrische Größe des Messstromes die Stromstärke des Messstromes sein.

Die Bezugselektrode kann insbesondere eine große Kontaktfläche aufweisen, um den Hautwiderstand an der Bezugsstelle gering zu halten.

Als Bezugselektrode kann insbesondere eine sogenannte Handelektrode verwendet werden. Eine derartige Handelektrode ist ein, insbesondere kreiszylinderförmiger, elektrisch leitfähiger Körper, welcher von dem Probanden in einer Hand gehalten wird. Durch die große Kontaktfläche zwischen der Handelektrode und der Hand des Probanden ist der elektrische Widerstand wesentlich geringer als der Hautwiderstand bei der im Wesentlichen punktförmigen Messelektrode 3.

Alternativ kann die Bezugselektrode auch an dem Probanden befestigt werden, beispielsweise durch ein Ankleben oder Anzurren.

Der Widerstand des Strompfades innerhalb des Körpers kann insbesondere dadurch im Wesentlichen konstant gehalten werden, indem die Bezugsstelle an einer ersten Extremität des Körpers des Probanden, und die vorgegebenen Messstellen an einer zweiten Extremität des Körpers des Probanden angeordnet ist.

Die vorgegebene Messstelle ist jene Stelle der Haut des Probanden, an welcher der elektrische Hautwiderstand gemessen wird und die hierfür auch mit der Messelektrode 3 in Kontakt gebracht wird. Besonders bevorzugt kann vorgesehen sein, dass die Messelektrode 3 bei einer Messreihe nacheinander mit einer Vielzahl an unterschiedlichen vorgegebenen Messstellen der Haut des Probanden in Kontakt gebracht wird.

Die vorgegebenen Messstellen können insbesondere Akkupunkturpunkte des Körpers des Probanden sein. Die Vermessung der Hautwiderstände an den Akkupunkturpunkten kann insbesondere für eine Elektroakkupunktur, insbesondere die Elektroakkupunktur nach Voll, erfolgen, gemäß welcher Krankheiten oder Allergien des Probanden durch die Hautwiderstände an den Akkupunkturpunkten feststellbar sind.

Weiters ist eine Anordnung zum Messen eines elektrischen Hautwiderstandes umfassend eine Vorrichtung 1, die Auswerteeinheit und die Bezugselektrode vorgesehen, wobei die Auswerteeinheit sowohl mit der Messelektrode 3 der Vorrichtung 1 als auch mit der Bezugselektrode schaltungstechnisch, insbesondere mittels elektrischen Kabeln 7, verbunden ist.

Besonders bevorzugt kann vorgesehen sein, dass der Kraftaufnehmer 4 auf die Messelektrode 3 einwirkende Kräfte in drei Koordinaten misst. Bei dem Verfahren kann vorgesehen sein, dass die auf die Messelektrode 3 einwirkenden Kräfte von dem Kraftaufnehmer 4 in drei Koordinaten gemessen werden. Die drei Koordinaten können insbesondere drei orthogonal zueinander sein. Durch die Messung der Kräfte in drei Koordinaten kann im Wesentlichen unabhängig von der Orientierung der Messelektrode 3 zu der Haut der Absolutwert der auf die Messelektrode 3 einwirkenden Kraft ermittelt werden. Diese auf die Messelektrode 3 einwirkenden Kraft kann insbesondere im Wesentlichen der Anpresskraft entsprechen.

Weiters kann vorgesehen sein, dass die Messelektrode 3 eine kugelförmige Spitze 5 aufweist. Durch die kugelförmige Spitze 5 bildet sich im Wesentlichen unabhängig von der Orientierung die gleiche Kontaktfläche zwischen der Spitze 5 und der Haut aus, sodass die Vorrichtung 1 von der Haut aus gesehen in einem großen Raumwinkelbereiche gedreht werden kann, und die Größe und Form der Kontaktfläche lediglich von der Anpresskraft und nicht von der Orientierung abhängt. Hierbei kann die Messelektrode 3 insbesondere einen sich zu der Spitze 5 hin verjüngenden Steg aufweisen, wobei an einem ersten Ende des Steges die kugelförmige Spitze 5 angeordnet ist. Die kugelförmige Spitze 5 kann gegenüber dem ersten Ende des Steges als Verdickung ausgebildet sein, sodass ein ausschließlicher Kontakt der Haut mit der Spitze 5 selbst bei einem flachen Anstellwinkel der Vorrichtung 1 gewährleistet ist.

Die Messelektrode 3 kann auch eine nicht kugelförmige Spitze 5 aufweisen. Allerdings können dann Messfehler dadurch entstehen, dass die Form der Kontaktfläche mit der Orientierung der Vorrichtung variiert.

Weiters kann vorgesehen sein, dass die Messelektrode 3 über den Kraftaufnehmer 4 mit dem Handgriff 2 verbunden ist. Bevorzugt kann der Kraftaufnehmer 4 zwischen dem Handgriff 2 und der Messelektrode 3 angeordnet sein. Besonders bevorzugt kann die Messelektrode 3 direkt an dem Kraftaufnehmer 4 befestigt sein. Durch diese Anordnung kann der Kraftaufnehmer 4 die gesamte auf die Messelektrode 3 einwirkende Kraft direkt aufnehmen, wodurch auf aufwendige Extrapolationen oder Kalibrierungen verzichtet werden kann. Die elektrische Leitung von der Messelektrode 3 kann insbesondere an dem Kraftaufnehmer 4 vorbei führen.

Alternativ kann der Kraftaufnehmer 4 die auf die Messelektrode 3 ausgeübte Kraft indirekt messen, insbesondere über eine Verformung des Handgriffes 2.

Besonders bevorzugt kann vorgesehen sein, dass der Kraftaufnehmer 4 ein optischer Kraftaufnehmer 4 ist. Der optische Kraftaufnehmer 4 weist bevorzugt eine Lichtquelle, einen, insbesondere ortsauflösenden, Lichtsensor, einen verformbaren Körper sowie eine reflektierende Oberfläche auf. Eine Verformung des verformbaren Körpers bewirkt eine Positionsänderung oder Verformung der von der Lichtquelle angeleuchteten reflektierenden Oberfläche, wobei die Positionsänderung oder Verformung der reflektierenden Oberfläche durch den Lichtsensor messbar ist. Vorteilhaft an einem derartigen Kraftaufnehmer 4 ist, dass diese sehr widerstandsfähig gegenüber schlagartigen Belastungen sind, da der die elektronischen Komponenten mit dem verformbaren Körper nicht in Kontakt sind. Dies ist aufgrund der Form der Vorrichtung 1 ein wesentlicher Vorteil, da diese häufig herab fallen und dabei zuerst auf der Messelektrode 3 landen. Durch die Verwendung eines optischen Kraftaufnehmers 4 kann ist die Vorrichtung 1 weniger anfällig gegenüber durch ein Herabfallen bewirkte Beschädigungen.

Insbesondere kann vorgesehen sein, dass die Messelektrode, insbesondere an einem der Spitze 5 gegenüberliegenden Ende, an dem verformbaren Körper, bevorzugt direkt, befestigt ist.

Insbesondere kann vorgesehen sein, dass der optische Kraftaufnehmer 4 einen verformbaren Körper in Form einer Kuppel aufweist, wobei zumindest Teile des verformbaren Körpers die reflektierende Oberfläche ausbilden, und dass die Lichtquelle und der Lichtsensor innerhalb der Kuppel angeordnet sind. Ein derartiger optischer Kraftaufnehmer 4 kann in einer besonders kompakten Bauweise realisiert werden, wodurch dieser für die Verwendung bei der Vorrichtung 1 besonders gut geeignet ist.

Alternativ kann vorgesehen sein, dass der Kraftaufnehmer 4 Piezoelemente umfasst.

Weiters kann vorgesehen sein, dass der Kraftaufnehmer 4 Dehnmessstreifen aufweist.

Damit die Auswerteeinheit den Hautwiderstand ermitteln kann werden Messdaten bezüglich der vom Kraftaufnehmer 4 gemessen Kräfte an die Auswerteeinheit übermittelt.

Besonders bevorzugt kann vorgesehen sein, dass die Messdaten die Anpresskraft der Messelektrode 3 an der vorgegebenen Messstelle umfassen. Hierbei können die Messdaten den Absolutwert der Anpresskraft umfassen. Weiter können die Messdaten die Richtung der Anpresskraft umfassen.

Weiters kann vorgesehen sein, dass der Kraftaufnehmer 4 zumindest mittelbar die Messdaten umfassend die Anpresskraft der Messelektrode 3 erstellt, dass die Auswerteeinheit ausgebildet ist die elektrische Größe des zwischen der Bezugselektrode und der Messelektrode 3 fließenden Messstromes zu messen und anhand der elektrischen Größe des Messstromes und unter Berücksichtigung der Messdaten den elektrischen Hautwiderstand des Probanden bei einer Referenzanpresskraft zu ermitteln.

Dass der Kraftaufnehmer 4 zumindest mittelbar die Messdaten erstellt bedeutet, dass der Kraftaufnehmer 4 unmittelbar die Messdaten erstellen kann, wobei die Messdaten dann dem Messsignal des Kraftaufnehmers 4 entsprechen, aber dass auch vorgesehen sein kann, dass der Kraftaufnehmer 4 den gemessenen Kräften in den wenigstens zwei Koordinaten entsprechende Messsignale ausgibt, und dass aus den Messsignalen die Messdaten berechnet werden.

Besonders bevorzugt kann vorgesehen sein, dass der Kraftaufnehmer 4 schaltungstechnisch mit einer, insbesondere im Handgriff 2 angeordnete, Signalverarbeitungselektronik 6 verbunden ist. Hierbei kann vorgesehen sein, dass die Messsignale von dem Kraftaufnehmer 4 von einer, insbesondere im Handgriff 2 angeordneten, Signalverarbeitungselektronik 6 zu den Messdaten verarbeitet werden. Die Messdaten können insbesondere digitale Daten sein. Durch die Signalverarbeitungselektronik 6 können die Messsignale von dem Kraftaufnehmer 4 insbesondere in digitale Messdaten gewandelt werden, welche von der Auswerteeinheit leicht weiterverarbeitbar sind. Von der Signalverarbeitungselektronik 6 können insbesondere Signalleitungen in das elektrische Kabel 7 führen. In Fig. 1 ist die Vorrichtung 1 als Prinzipskizze stark vereinfach dargestellt, wobei die Pfeile den Informationsfluss bezüglich der Anpresskraft darstellen.

Weiters kann vorgesehen sein, dass aus den Messsignalen der Absolutwert der Anpresskraft, also die Größe des Vektors der gemessenen auf die Messelektrode einwirkenden Kraft, berechnet und als Messdaten ausgegeben werden.

Die Auswerteeinheit kann insbesondere ein elektronisches Gerät mit einem Speicher, einem Prozessor und einer Anzeigevorrichtung sein.

Die Auswerteeinheit ist dazu vorgesehen anhand der Messdaten und der gemessenen elektrischen Größe des Messstromes den Hautwiderstand des Probanden bei der Referenzanpresskraft an der wenigstens einen Messstelle zu ermitteln.

Durch die elektrische Größe des Messstromes kann der gesamte elektrische Widerstand zwischen der Bezugselektrode und der Messelektrode bestimmt werden, wobei in der Auswerteeinheit eingestellt werden kann, wie von dem gemessenen elektrischen Widerstand zwischen der Bezugselektrode und der Messelektrode der, allerdings von der Anpresskraft abhängige, Hautwiderstand berechnet werden kann.

Insbesondere kann vorgesehen sein, dass in der Auswerteeinheit die Art der Bezugselektrode und/oder ein zumindest ungefährer Abstand der vorgegebenen Bezugsstelle zu der vorgegebenen Messstelle eingestellt werden kann.

Sofern der Kraftaufnehmer 4 die Kräfte in lediglich zwei Koordinaten misst können in der Auswerteeinheit bevorzugt weitere Größen, beispielsweise der Anstellwinkel der Vorrichtung 1 zu der Haut, eingestellt werden, welche es ermöglich von den Kräften in zwei Koordinaten auf die Anpresskraft zu schließen.

Diese Einstellungen können insbesondere in der Auswerteeinheit vorab gespeichert sein.

Weiters können die ermittelten elektrische Hautwiderstände bei der Referenzanpresskraft als Messergebnisse abgespeichert werden.

Weiters kann vorgesehen sein, dass in der Auswerteeinheit die Referenzanpresskraft eingestellt wird. Die Referenzanpresskraft ist hierbei jene vorgegebene einheitliche Anpresskraft für die der elektrische Hautwiderstand als Messergebnis verwendet werden kann.

Der elektrische Hautwiderstand eines Probanden bei einer Referenzanpresskraft an der wenigstens einen Messstelle kann von der Auswerteeinheit auf unterschiedliche Weise ermittelt werden.

Es kann vorgesehen sein, dass von der Auswerteeinheit ermittelt wird, wann die aus den Messdaten hervorgehende Anpresskraft zumindest im Wesentlichen die Referenzanpresskraft ist. Die Anpresskraft kann hierbei direkt in den Messdaten bereits enthalten sein und/oder anhand den Messdaten von der Auswerteeinheit bestimmbar sein. Zumindest im Wesentlichen bedeutet, dass ein Bereich um die Referenzanpresskraft vorgegeben werden kann, und dass die Auswerteeinheit ermittelt, wann die Anpresskraft in diesem zumindest vorgebbaren Bereich um die Referenzanpresskraft ist. Der vorgebbare Bereich kann auch als Anpresskrafttoleranzbereich bezeichnet werden. Dadurch kann bestimmt werden, wann die Anpresskraft zumindest im Wesentlichen die Referenzanpresskraft ist, wobei der in diesem Moment gemessene elektrische Hautwiderstand dem elektrischen Hautwiderstand bei der Referenzanpresskraft entspricht.

Hierbei kann vorgesehen sein, dass die Auswerteeinheit dem Benutzer anzeigt, wenn die Anpresskraft zumindest im Wesentlichen die Referenzanpresskraft ist. Der Benutzer kann dann den momentanen elektrischen Hautwiderstand als elektrischer Hautwiderstand des Probanden bei der Referenzanpresskraft verwendet.

Weiters kann vorgesehen sein, dass die Auswerteeinheit, wenn die Anpresskraft zumindest im Wesentlichen die Referenzanpresskraft ist den momentanen elektrischen Hautwiderstand als Messergebnis für die Messstelle abspeichert.

Insbesondere kann vorgesehen sein, dass die Anpresskraft für einen vorgebbaren Zeitraum zumindest im Wesentlichen die Referenzanpresskraft entsprechen muss, damit die Auswerteeinheit den momentanen elektrischen Hautwiderstand als Messergebnis für die Messstelle abspeichert.

Weiters kann vorgesehen sein, dass der elektrische Hautwiderstand bei der Referenzanpresskraft von der Auswerteeinheit anhand der elektrischen Größe des Messstromes und der Anpresskraft der Messelektrode 3 berechnet wird. Hierbei kann vorgesehen sein, dass eine Korrekturfunktion des elektrischen Hautwiderstandes als Funktion der Anpresskraft vorgegeben wird. Diese Korrekturfunktion kann insbesondere durch Kalibrierungen oder vorangehende Versuche bestimmt werden. Dadurch kann die Auswerteeinheit bei einer nicht der Referenzanpresskraft entsprechenden Anpresskraft von dem gemessenen elektrischen Hautwiderstand den elektrischen Hautwiderstand bei der Referenzanpresskraft berechnen. Dadurch ist es nicht mehr erforderlich, dass der Benutzer tatsächlich eine der Referenzanpresskraft entsprechende Anpresskraft ausüben muss, und dennoch ein elektrischer Hautwiderstand bei der Referenzanpresskraft ermittelt werden kann. Dadurch kann die Durchführung der Messung erheblich beschleunigt werden.

Insbesondere kann vorgesehen sein, dass an der wenigstens einen vorgegebenen Messstelle über einen vorgegebenen ersten Messzeitraum eine Vielzahl an ersten Messdaten aufgenommen wird und einen zeitlichen Verlauf der Anpresskraft ermittelt wird, und dass ein zeitlicher Verlauf der elektrischen Größe des Messstromes in dem vorgegebenen ersten Messzeitraum mit dem zeitlichen Verlauf der Anpresskraft verglichen wird, um den elektrischen Hautwiderstand bei der Referenzanpresskraft zu ermitteln. Hierbei kann vorgesehen sein, dass aus dem zeitlichen Verlauf der elektrischen Größe ein zeitlicher Verlauf des elektrischen Hautwiderstandes errechnet wird. Dadurch, dass nicht nur ein einzelner Wert der elektrischen Größe des Messstromes und der Anpresskraft für die Auswertung verwendet wird, sondern mehrere Werte über einen zeitlichen Verlauf, kann die Genauigkeit der Messung nochmals verbessert werden.

Insbesondere kann für mehrere Zeitpunkte in dem vorgegebenen ersten Messzeitraum der elektrische Hautwiderstand bei der Referenzanpresskraft berechnet werden, wobei die berechneten elektrischen Hautwiderstände bei der Referenzanpresskraft innerhalb des ersten Messzeitraumes gemittelt und als elektrische Hautwiderstand bei der Referenzanpresskraft für den ersten Messzeitraum ausgegeben werden.

Weiters kann vorgesehen sein, dass durch den Vergleich des zeitlichen Verlaufes der elektrischen Größe und der Anpresskraft eine Funktion des elektrischen Hautwiderstandes in Abhängigkeit der Anpresskraft erstellt wird. Hierbei kann der elektrische Hautwiderstand von einer Vielzahl an Faktoren abhängen, beispielsweise der Größe der Kontaktfläche, eine druckabhängige elektrische Leitfähigkeit der Haut an sich, der Feuchtigkeit der Haut und/oder der Aktivität der Schweißdrüsen. Hierbei können unterschiedliche Faktoren des elektrischen Hautwiderstandes unterschiedlich auf eine Änderung der Anpresskraft reagieren. Durch die Bestimmung der Funktion des elektrischen Hautwiderstandes in Abhängigkeit der Anpresskraft können daher zusätzliche Informationen über die einzelnen Faktoren gewonnen werden, welche unter Umständen eine genauere Diagnose ermöglichen.

Weiters kann vorgesehen sein, dass Faktoren, welche als Störfaktor angesehen werden, insbesondere die Feuchtigkeit der Haut, bei der Bestimmung des elektrischen Hautwiderstandes bei der Referenzanpresskraft berücksichtigt werden können, um einen um diesen Faktor korrigierten elektrischen Hautwiderstand auszugeben.

Es kann vorgesehen sein, dass die Auswerteeinheit in der Anzeigevorrichtung die Anpresskraft und/oder den elektrischen Hautwiderstand und/oder den elektrischen Hautwiderstandes bei der Referenzanpresskraft anzeigt.

Weiters kann vorgesehen sein, dass die Auswerteeinheit die Anpresskraft und/oder den elektrischen Hautwiderstand und/oder den elektrischen Hautwiderstandes bei der Referenzanpresskraft und/oder zeitliche Verläufe von diesen, insbesondere der jeweiligen Messstellen zugeordnet, abspeichert.

Bei dem Verfahren kann vorgesehen sein, dass der elektrische Hautwiderstand bei der Referenzanpresskraft bei einer Vielzahl an verschiedenen vorgesehenen Messstellen ermittelt wird.

Insbesondere kann vorgesehen sein, dass an den vorgesehenen Messstellen gemäß einer vorgegebenen Reihung der elektrische Hautwiderstand bei der Referenzanpresskraft ermittelt wird, und die ermittelten Messergebnisse insbesondere abgespeichert werden.

Insbesondere kann vorgesehen sein, dass einer spezifischen vorgesehenen Messstelle ein spezifisches Messergebnis zuordenbar abgespeichert wird. insbesondere kann vorgesehen sein, dass die Auswerteeinheit ein Signal an den Benutzer ausgibt, wenn das Messergebnis für die momentane Messstelle abgespeichert wurde, damit der Benutzer mit der Vorrichtung 1 zu der nächsten vorgesehenen Messstelle wechseln kann. Das Signal kann insbesondere über die Anzeigevorrichtung und/oder eine eigen, bevorzugt akustische, Signalvorrichtung ausgegebenen werden. Dadurch kann dem Benutzer angezeigt werden, dass er zu der nächsten Messstelle wechseln kann.

Weiters kann vorgesehen sein, dass die Auswerteeinheit anhand der Messdaten und/oder der elektrischen Größe erkennt, wann eine Messung anfängt. Insbesondere kann das dadurch erfolgen, dass die Auswerteeinheit ein Vorhandensein einer Anpresskraft oder eines Messstromes als Bedingung erkennen kann, ob die Messelektrode 3 mit einer Haut in Berührung ist.

Weiters kann vorgesehen sein, dass die Positionen der für die Messung vorgesehenen Messstellen an der Anzeigevorrichtung dargestellt werden. Insbesondere kann die Position der aktuellen vorgesehenen Messstelle und/oder der nächsten vorgesehenen Messstelle angezeigt werden. Dadurch kann das Verfahren auch von geringfügig eingeschulten Personal zuverlässig und reproduzierbar durchgeführt werden.

Weiters ist vorgesehen, dass der Kraftaufnehmer 4 als Beschleunigungssensor für die Vorrichtung 1 verwendet wird. Hierbei kann vorgesehen sein, dass der Kraftaufnehmer 4 als Beschleunigungssensor verwendet werden kann, wenn keine Anpresskraft vorhanden ist, da dann die von dem Kraftaufnehmer 4 gemessene Kraft der Gewichtskraft und einer durch eine Beschleunigung ausgeübte Kraft der Messelektrode 3 entspricht. Dadurch kann insbesondere seitens der Auswerteeinheit erkannt werden, wenn der Benutzer die Vorrichtung 1 aufnimmt.

Weiters kann vorgesehen sein, dass die Auswerteinheit anhand der Messdaten detektiert, ob der Kraftaufnehmer 4 funktionstüchtig ist.

Weiters kann vorgesehen sein, dass die Auswerteeinheit einen Ausbildungsmodus aufweist. In dem Ausbildungsmodus kann insbesondere vorgesehen sein, dass die Anpresskraft angezeigt wird. Dadurch können unerfahrene Benutzer mit der Auswerteeinheit üben eine Anpresskraft aufzubringen, welche zumindest in der Nähe der Referenzanpresskraft ist.

## Patentansprüche

1. Vorrichtung (1) zum Messen eines elektrischen Hautwiderstandes, umfassend einen stiftförmigen Handgriff (2), eine an einem Ende des Handgriffes (2) angeordnete im Wesentlichen punktförmige Messelektrode (3) und einen Kraftaufnehmer (4), **dadurch gekennzeichnet, dass** der Kraftaufnehmer (4) auf die Messelektrode (3) einwirkende Kräfte in wenigstens zwei Koordinaten misst, dass die Messelektrode (3) eine, an einem ersten Ende eines Steges angeordnete kugelförmige Spitze (5) aufweist, und dass die kugelförmige Spitze (5) gegenüber dem ersten Ende des Steges als Verdickung ausgebildet ist.

2. Vorrichtung (1) nach Anspruch 1, **dadurch gekennzeichnet, dass** der Kraftaufnehmer (4) auf die Messelektrode (3) einwirkende Kräfte in drei Koordinaten misst.

3. Vorrichtung (1) nach Anspruch 1 oder 2, **dadurch gekennzeichnet, dass** die Messelektrode (3) über den Kraftaufnehmer (4) mit dem Handgriff (2) verbunden ist.

4. Vorrichtung (1) nach einem der Ansprüche 1 bis 3, **dadurch gekennzeichnet, dass** der Kraftaufnehmer (4) ein optischer Kraftaufnehmer (4) ist.

5. Vorrichtung (1) nach einem der Ansprüche 1 bis 4, **dadurch gekennzeichnet, dass** der Kraftaufnehmer (4) schaltungstechnisch mit einer, insbesondere im Handgriff (2) angeordnete, Signalverarbeitungselektronik (6) verbunden ist.

6. Anordnung zum Messen eines elektrischen Hautwiderstandes umfassend eine Vorrichtung (1) nach einem der Ansprüche 1 bis 5, eine Auswerteeinheit und eine Bezugselektrode, wobei die Auswerteeinheit sowohl mit der Messelektrode (3) der Vorrichtung (1) als auch mit der Bezugselektrode schaltungstechnisch verbunden ist.

7. Anordnung nach Anspruch 6, **dadurch gekennzeichnet, dass** der Kraftaufnehmer (4) unmittelbar Messdaten umfassend eine Anpresskraft der Messelektrode (3) erstellt, oder dass der Kraftaufnehmer (4) den gemessenen Kräften in den wenigstens zwei Koordinaten entsprechende Messsignale ausgibt aus welchen die Messdaten umfassend eine Anpresskraft der Messelektrode (3) berechenbar sind, und dass die Auswerteeinheit ausgebildet ist eine elektrische Größe eines zwischen der Bezugselektrode und der Messelektrode (3) fließenden Messstromes zu messen und anhand der elektrischen Größe des Messstromes und unter Berücksichtigung der Messdaten den elektrischen Hautwiderstand eines Probanden bei einer Referenzanpresskraft zu ermitteln.

8. Verfahren zum Messen eines elektrischen Hautwiderstandes eines Probanden, wobei eine Bezugselektrode an einer vorgegebenen Bezugsstelle von dem Probanden gehalten oder an dem Probanden befestigt wird, wobei eine an einem Ende eines stiftförmigen Handgriffes (2) angeordnete im Wesentlichen punktförmige Messelektrode (3) an wenigstens einer vorgegebenen Messstelle mit einer Anpresskraft gegen die Haut des Probanden gedrückt wird, **dadurch gekennzeichnet, dass** die Messelektrode (3) eine, an einem ersten Ende eines Steges angeordnete kugelförmige Spitze (5) aufweist, wobei die kugelförmige Spitze (5) gegenüber dem ersten Ende des Steges als Verdickung ausgebildet ist, wobei auf die Messelektrode (3) einwirkende Kräfte in wenigsten zwei Koordinaten von einem Kraftaufnehmer (4) gemessen und als Messdaten an eine Auswerteeinheit übermittelt werden, wobei ein Messstrom zwischen der Bezugselektrode und der Messelektrode (3) erzeugt wird, wobei von der Auswerteeinheit anhand einer elektrischen Größe des Messstromes und unter Berücksichtigung der Messdaten des Kraftaufnehmers (4) der elektrische Hautwiderstand eines Probanden bei einer Referenzanpresskraft an der wenigstens einen vorgegebenen Messstelle ermittelt wird.

9. Verfahren nach Anspruch 8, **dadurch gekennzeichnet, dass** die auf die Messelektrode (3) einwirkenden Kräfte von dem Kraftaufnehmer (4) in drei Koordinaten gemessen werden.

10. Verfahren nach Anspruch 8 oder 9, **dadurch gekennzeichnet, dass** die Messdaten die Anpresskraft der Messelektrode (3) an die vorgegebenen Messstelle umfassen.

11. Verfahren nach einem der Ansprüche 8 bis 10, **dadurch gekennzeichnet, dass** Messsignale von dem Kraftaufnehmer (4) von einer, insbesondere im Handgriff (2) angeordneten, Signalverarbeitungselektronik (6) zu den Messdaten verarbeitet werden.

12. Verfahren nach einem der Ansprüche 8 bis 11, **dadurch gekennzeichnet, dass** von der Auswerteeinheit ermittelt wird, wann die aus den Messdaten hervorgehende Anpresskraft zumindest im Wesentlichen die Referenzanpresskraft ist.

13. Verfahren nach einem der Ansprüche 8 bis 12, **dadurch gekennzeichnet, dass** der elektrische Hautwiderstand bei der Referenzanpresskraft von der Auswerteeinheit anhand der elektrischen Größe des Messstromes und der Anpresskraft der Messelektrode (3) berechnet wird.

14. Verfahren nach einem der Ansprüche 8 bis 12, **dadurch gekennzeichnet, dass** an der wenigstens einen vorgegebenen Messstelle über einen vorgegebenen ersten Messzeitraum eine Vielzahl an ersten Messdaten aufgenommen wird und einen zeitlichen Verlauf der Anpresskraft ermittelt wird, und dass ein zeitlicher Verlauf der elektrischen Größe des Messstromes in dem vorgegebenen ersten Messzeitraum mit dem zeitlichen Verlauf der Anpresskraft verglichen wird, um den elektrischen Hautwiderstand bei der Referenzanpresskraft zu ermitteln.

## Claims

1. Device (1) for measuring an electrical skin resistance, comprising a pin-shaped handle (2), a substantially point-shaped measuring electrode (3) arranged at one end of the handle (2) and a force transducer (4), **characterized in that** the force transducer (4) measures forces acting on the measuring electrode (3) in at least two coordinates, **in that** the measuring electrode (3) has a spherical tip (5) arranged at a first end of a web, and **in that** the spherical tip (5) is designed as a thickening relative to the first end of the web.

2. Device (1) according to claim 1, **characterized in that** the force transducer (4) measures forces acting on the measuring electrode (3) in three coordinates.

3. Device (1) according to claim 1 or 2, **characterized in that** the measuring electrode (3) is connected to the handle (2) via the force transducer (4).

4. Device (1) according to one of claims 1 to 3, **characterized in that** the force transducer (4) is an optical force transducer (4).

5. Device (1) according to one of claims 1 to 4, **characterized in that** the force transducer (4) is connected by circuitry to signal processing electronics (6), in particular arranged in the handle (2).

6. Arrangement for measuring an electrical skin resistance, comprising a device (1) according to one of claims 1 to 5, an evaluation unit and a reference electrode, wherein the evaluation unit is connected both to the measuring electrode (3) of the device (1) and to the reference electrode by circuitry.

7. Arrangement according to claim 6, **characterized in that** the force transducer (4) directly produces measurement data comprising a contact force of the measuring electrode (3), or **in that** the force transducer (4) outputs measurement signals corresponding to the measured forces in the at least two coordinates, from which the measurement data comprising a contact force of the measuring electrode (3) can be calculated, and **in that** the evaluation unit is designed to measure an electrical quantity of a measuring current flowing between the reference electrode and the measuring electrode (3) and to determine, on the basis of the electrical quantity of the measuring current and taking into account the measurement data, the electrical skin resistance of a test person at a reference contact pressure.

8. Method for measuring an electrical skin resistance of a test person, wherein a reference electrode is held at a predetermined reference point by the test person or is fastened to the test person, wherein a substantially point-shaped measuring electrode (3) arranged at one end of a pin-shaped handle (2) is pressed with a contact force against the skin of the test person at at least one predetermined measuring point, **characterized in that** the measuring electrode (3) has a spherical tip (5) arranged at a first end of a web, wherein the spherical tip (5) is formed as a thickening relative to the first end of the web, wherein forces acting on the measuring electrode (3) are measured in at least two coordinates by a force transducer (4) and transmitted as measurement data to an evaluation unit, wherein a measuring current is generated between the reference electrode and the measuring electrode (3), wherein the electrical skin resistance of a test person is determined by the evaluation unit on the basis of an electrical quantity of the measuring current and by taking into account the measurement data of the force transducer (4) at a reference contact pressure at the at least one predetermined measuring point.

9. Method according to claim 8, **characterized in that** the forces acting on the measuring electrode (3) are measured by the force transducer (4) in three coordinates.

10. Method according to claim 8 or 9, **characterized in that** the measurement data comprise the contact force of the measuring electrode (3) to the predetermined measuring point.

11. Method according to one of claims 8 to 10, **characterized in that** measurement signals from the force transducer (4) are processed into the measurement data by signal processing electronics (6) which are especially arranged in the handle (2).

12. Method according to one of claims 8 to 11, **characterized in that** the evaluation unit determines when the contact pressure resulting from the measurement data is at least essentially the reference contact pressure.

13. Method according to one of claims 8 to 12, **characterized in that** the electrical skin resistance at the reference contact pressure is calculated by the evaluation unit on the basis of the electrical quantity of the measuring current and the contact pressure of the measuring electrode (3).

14. Method according to one of claims 8 to 12, **characterized in that** a plurality of first measurement data is recorded at the at least one predetermined measuring point over a predetermined first measurement period and a temporal course of the contact force is determined, and **in that** a temporal course of the electrical quantity of the measuring current in the predetermined first measurement period is compared with the temporal course of the contact force in order to determine the electrical skin resistance at the reference contact force.

## Revendications

1. Dispositif (1) pour mesurer la résistance électrique de la peau, comprenant une poignée (2) en forme de crayon, une électrode de mesure (3) sensiblement en forme de point disposée à l'extrémité de la poignée (2) et un capteur de force (4), **caractérisé en ce que** le capteur de force (4) mesure la force agissant sur l'électrode de mesure (3) sur au moins deux axes de coordonnées, **en ce que** l'électrode de mesure (3) présente une pointe sphérique (5) disposée à une première extrémité d'une tige et **en ce que** la pointe sphérique (5) est conformée comme un épaississement par rapport à la première extrémité de la tige.

2. Dispositif (1) selon la revendication 1, **caractérisé en ce que** le capteur de force (4) mesure les forces agissant sur l'électrode de mesure (3) sur trois axes de coordonnées.

3. Dispositif (1) selon la revendication 1 ou 2, **caractérisé en ce que** l'électrode de mesure (3) est reliée à la poignée (2) par l'intermédiaire du capteur de force (4).

4. Dispositif (1) selon l'une des revendications 1 à 3, **caractérisé en ce que** le capteur de force (4) est un capteur de force optique (4).

5. Dispositif (1) selon l'une des revendications 1 à 4, **caractérisé en ce que** le capteur de force (4) est connecté en circuit à une électronique de traitement des signaux (6), disposée en particulier dans la poignée (2).

6. Disposition pour mesurer la résistance électrique de la peau comprenant un dispositif (1) selon l'une des revendications 1 à 5, une unité d'analyse et une électrode de référence, l'unité d'analyse étant connectée en circuit aussi bien avec l'électrode de mesure (3) du dispositif (1) qu'avec l'électrode de référence.

7. Disposition selon la revendication 6, **caractérisé en ce que** le capteur de force (4) produit directement des données de mesure comprenant une force d'appui de l'électrode de mesure (3) ou **en ce que** le capteur de force (4) émet en sortie des signaux de mesure correspondant aux forces mesurées sur les au moins deux axes de coordonnées à partir desquels les données de mesure comprenant une force d'appui de l'électrode de mesure (3) peuvent être calculées, et **en ce que** l'unité d'analyse est conformée pour déterminer une grandeur électrique d'un courant circulant entre l'électrode de référence et l'électrode de mesure (3) et, à partir de la grandeur électrique du courant de mesure et en tenant compte des données de mesure, la résistance électrique de la peau d'un sujet à une force d'appui de référence.

8. Procédé pour mesurer la résistance électrique de la peau d'un sujet, dans lequel une électrode de référence est tenue par le sujet ou fixée sur le sujet en un point de référence prédéterminé, une électrode de mesure (3) disposée à l'extrémité d'une poignée (2) en forme de crayon étant appuyée contre la peau du sujet en au moins un point de mesure prédéterminé à une certaine force d'appui, **caractérisé en ce que** l'électrode de mesure (3) présente une pointe sphérique (5) disposée à une première extrémité d'une tige et **en ce que** la pointe sphérique (5) est conformée comme un épaississement par rapport à la première extrémité de la tige, les forces qui agissent sur l'électrode de mesure (3) étant mesurées par un capteur de force (4) sur au moins deux axes de coordonnées et transmises comme données de mesure à une unité d'analyse, un courant de mesure entre l'électrode de référence et l'électrode de mesure (3) étant créé, l'unité d'analyse déterminant, à partir de la grandeur électrique du courant de mesure et en tenant compte des données de mesure, la résistance électrique de la peau d'un sujet à une force d'appui de référence à l'au moins un point de mesure prédéterminé.

9. Procédé selon la revendication 8, **caractérisé en ce que** les forces agissant sur l'électrode de mesure (3) sont mesurées par le capteur de force (4) sur trois axes de coordonnées.

10. Procédé selon la revendication 8 ou 9, **caractérisé en ce que** les données de mesure comprennent la force d'appui de l'électrode de mesure (3) au point de mesure prédéterminé.

11. Procédé selon l'une des revendications 8 à 10, **caractérisé en ce que** les signaux de mesure du capteur de force (4) sont traités par une électronique de traitement des signaux (6), disposée en particulier dans la poignée (2), pour produire les données de mesure.

12. Procédé selon l'une des revendications 8 à 11, **caractérisé en ce que** l'unité d'analyse détermine quand la force d'appui indiquée dans les données de mesure est au moins sensiblement la force d'appui de référence.

13. Procédé selon l'une des revendications 8 à 12, **caractérisé en ce que** la résistance électrique de la peau à la force d'appui de référence est calculée par l'unité d'analyse à l'aide de la grandeur électrique du courant de mesure et de la force d'appui de l'électrode de mesure (3).

14. Procédé selon l'une des revendications 8 à 12, **caractérisé en ce qu'**un grand nombre de premières données de mesure sont captées à l'au moins un point de mesure prédéterminé sur un premier intervalle de temps de mesure prédéterminé et une évolution dans le temps de la force d'appui est déterminée, et **en ce qu'**une évolution dans le temps de la grandeur électrique du courant de mesure sur le premier intervalle de temps de mesure prédéterminé est comparé à l'évolution dans le temps de la force d'appui pour déterminer la résistance électrique de la peau à la force d'appui de référence.
